# EUROPEAN PATENT APPLICATION

(11) **EP 1 508 342 A1**
(43) Date of publication of application: **23.02.2005**
(21) Application number: 04017160.5
(22) Date of filing: 21.07.2004
(51) Int. Cl.: A61L 9/01, A61L 9/013, B65F 7/00

(54) **Deodourising biocidal compositions**

(30) Priority: 20.08.2003 GB 0319537
(71) Applicant: Allied Bio Corporation Limited, Oldham, Lancashire OL1 3DN (GB)
(72) Inventor: Latham, George, Springhead, Oldham OL4 5UB (GB); Peel, Adrian, Royton OL2 6EU (GB)
(74) Representative: Appleton, Ben

(57) **Abstract**

The invention provides a sanitary composition for use in an airspace within a container, THE sanitary composition comprising a filler and at least two oils, each oil having a sanitary property. The invention further provides a method of treating an airspace within a container.

## Description

### Field of the Invention

This invention relates to sanitary compositions and methods of treating an airspace within a container.

### Background to the Invention

It is well known to use sterilising agents which form sulphur-dioxide to sterilise enclosed spaces. Such agents have been used in a wide variety of applications including sterilisation of fermentation bins and sanitary bins.

In the case of fermentation bins, granules or tablets of the agent are dissolved rapidly in water in the fermentation bin, which quickly releases large quantities of sulphur-dioxide for fast sterilisation.

In the case of sanitary bins, where there is only a small amount of moisture in the air and/or materials inside the bin, it is usual to add a portion of the sterilising agent in powder or granule form. The powder or granules have a large surface area which enables activation by the available moisture to form a sufficient amount of sulphur-dioxide for sterilisation.

In each case, the basic need is to be able to release large quantities of sulphur-dioxide at the appropriate time in order to prevent undue build up of microorganisms within the airspace. For this reason, granules are preferred which have a large surface area to a volume ratio, which enables activation by available moisture as quickly as possible.

However, in many applications, including sanitary bins, the build up microorganisms is relatively slow, and the use of sulphur-dioxide liberating compounds has more disadvantages than advantages. Disadvantages include some sulphur-dioxide liberating compounds being hazardous to the health of manufacturers, users and persons placing the tablets or granules in the container to be sterilised.

In many situations it is not necessary to sterilise an airspace such as a sanitary bin, sharps bin etc but merely to provide biostatic properties in order to prevent undue build up of microorganisms.

The use of sulphur-dioxide liberating compounds and other sterilising agents, although effective at killing bacteria and other microorganisms, does not always prevent unpleasant odours from emanating from the container in which the contaminated materials and sterilising agents are placed.

Furthermore, the use of the granules creates further problems in granule dust, dispersion which may be inhaled, and cause harmful effects in the user.

It would therefore be advantageous to provide a sanitary composition which comprises substantially no ingredients which are hazardous to a user's health, and preferably comprises natural ingredients which incorporate deodorising, odour masking and/or biostatic or biocidal effects.

It would furthermore be advantageous to provide a sanitary composition in a tablet having a substantially three-dimensional structure such that degradation of the tablet is relatively slow, and which does not produce dust in the air space of the container or out of a container to be treated.

It has been surprisingly found by the inventor that provision of a sanitary composition which includes a bulk filler, and two natural or synthetic oils having sanitary properties, provides a tablet which includes no harmful ingredients to a user's health and may provide combined deodorising and biocidal/biostatic action depending on the different oils used.

It is therefore an object of preferred embodiments of the present invention to overcome or mitigate at least one problem of the prior art, whether expressly disclosed herein or not.

### Summary of the Invention

According to a first aspect of the invention there is provided a sanitary composition for use in an airspace within a container, the sanitary composition comprising a filler and at least two oils, each oil having a sanitary property.

The at least two oils may be natural oils, synthetic oils or a combination of at least one natural oil and at least one synthetic oil.

Suitably the sanitary composition is in the form of a solid tablet.

By "tablet" we mean a solid having substantial three-dimensional structure, preferably having a thickness of at least 2mm, more preferably at least 5mm, still more preferably at least 8mm and most preferably at least 10mm. Suitably the tablet comprises consolidated powder or granules of solid material.

By "sanitary effect" we mean that the oil(s) effects a biocidal, biostatic, deodorising, odour neutralising or odour-masking effect.

Suitably each tablet is supplied in its own sealed space. For example it may be individually wrapped or provided in "blister pack" form.

Alternatively tablets may be packaged together, preferably in a sealed container containing, separately, a hygroscopic agent (for example silica gel) able preferentially to absorb atmospheric moisture, and so prevent premature activation of any ingredient within the tablet. Such a hygroscopic agent may also be employed when each tablet is supplied in its own sealed space. Thus, the tablets may be kept in an inactive form until needed, prolonging their shelf-life and subsequent utility.

Alternatively the composition may be in the form of a solid film, preferably having a thickness of less than 2mm, more preferably less than 1mm.

The composition may be in the form of a viscous liquid, xerogel (a hydrogel in the substantially dehydrated state) or a hydrogel.

The oil is preferably an aromatic oil and is preferably a perfumed oil.

Suitable natural oils include tea-tree oil, orange tree oil, citrus oil, jojoba oil, aloe vera oil, apricot oil, camellia oil, castor oil, coconut oil, rosehip oil, ambergris, apple blossom oil, jasmine oil, lilac oil, lavender oil, magnolia oil, orange blossom oil, oil of evening primrose, rose oil, wisteria oil, mentholyptus oil, eucalyptus oil.

Preferably the composition comprises at least one oil which exhibits biocidal or biostatic properties, including for example, tea tree oil, which is preferred.

Thus preferably the composition comprises tea-tree oil as one of the oils, and at least one further oil. Suitably at least one further oil is a perfumed oil, arranged to at least partially evaporate from the composition during use, into the airspace into which the composition is placed, and render the airspace fragrant. Preferably the further oil is a plant derived oil and more preferably is a citrus oil such as orange oil or lemon oil. It has been found that a combination of tea-tree oil as a biocidal oil and a citrus oil as a perfumed oil effects good biocidal action in an airspace, and in which the citrus oil effects dispersal of a pleasant aroma, which may mask the tea-tree oil.

Suitably the tea-tree oil is present in an amount of at least 0.1% w/w of the total weight of the composition, preferably at least 0.25% w/w and preferably at least 0.5% w/w.

Suitably the citrus oil is present in an amount of at least 0.5% w/w, preferably at least 0.75% w/w and more preferably at least 1.0% w/w.

Suitable synthetic oils include any synthetic or synthesized equivalents of natural oils, preferably of those described hereinabove, for example, synthetic citrus oil, including orange oil, and synthetic tea-tree oil.

Preferably the composition further comprises a hygroscopic compound.

Preferably the hygroscopic compound is a hygroscopic alkylbenzenesulphonate, or dialkylbenzenesulphonate. However, other types of hygroscopic material may be used.

A preferred dialkylbenzenesulphonate is di-isopropylbenzenesulphonate.

Preferably the hygroscopic compound comprises at least 0.5%wt of the total weight of the sanitary composition, more preferably at least 1%wt.

Preferably the hygroscopic compound comprises no more than 5%wt of the total weight of the sanitary composition, more preferably no more than 2.5% wt.

Thus a preferred range for the hygroscopic compound is 1-2.5%wt of the total weight of the sanitary composition.

The block should be such that it produces an insubstantial amount, and preferably no, harmful dust when inserted into the airspace.

Preferably the composition further comprises a lubricant, such as a polyglycol compound, a metallic stearate, stearic acid, colloidal silicas or talc, for example.

Preferably the sanitary composition comprises a polyglycol compound, more preferably a polyethylene glycol compound.

Suitably the polyglycol compound comprises at least 0.5%wt of the total weight of the sanitary composition, preferably at least 1%wt.

Preferably the polyglycol compound comprises no more than 10%wt of the total weight of the sanitary composition, more preferably no more than 5% wt.

Suitably the composition comprises a compound capable of absorbing moisture.

The compound capable of absorbing moisture may comprise an absorbent polymer, such as a cellulosic polymer, including cotton for example.

The composition may include a sulphur-dioxide liberating compound.

Suitably the sulphur dioxide activating compound is a metabisulphite, preferably sodium metabisulphite or potassium metabisulphite.

The filler may comprise any natural or synthetic filler ingredient, such as cellulose, carboxymethyl cellulose, diphosphate containing compounds, lactose, lactose monohydrate, dicalcium phosphate, calcium sulphate and the like, for example.

Suitably the filler is present in an amount of at least 10% w/w of the total weight of the composition, more preferably at least 20% w/w and most preferably at least 25% w/w.

Preferably the composition comprises a disintegrant. Suitable disintegrants include starch, cross-linked carboxymethyl celluloses, cross-linked polyvinylpyrrolidones and sodium starch glycolates, for example. Preferred as a disintegrant is starch.

The disintegrant may also act as a filler, and vice versa.

Suitably the disintegrant is present in the composition in an amount of at least 20% w/w of the total weight of the composition, preferably at least 30% w/w and more preferably at least 40% w/w.

Preferably the disintegrant is starch.

The composition may comprise one or more further ingredients, such as salts, including sodium chloride, organic acids and/or their salts, buffers, solvents, colourants and the like, for example.

According to a second aspect of the invention there is provided a method of treating an airspace within a container, the method comprising locating a sanitary composition of the first aspect of the invention in the container.

Preferably the container is generally enclosed. Preferably the container is used for deposit or storage of contaminated, or more preferably biological materials, for example biological soils, microorganisms, or biological waste products.

Preferably the container is a sanitary bin.

Alternatively, the container may be a medical dressing container, a nappy bin, a used sharps bin, a post box, a body bag or a container used for the disposal or containment of any contaminated or, preferably, biological waste.

### Examples

For a better understanding of the invention the various aspects will now be described by way of example embodiment only.

### Example 1

A tablet of compressed granules was prepared using the following ingredients to form a sanitary composition of the invention:

| | **% weight of the total weight of the composition** |
|---|---|
| Polyethylene glycol 6000 | 2.00 |
| Sodium chloride | 20.00 |
| Cellulose (Avicel (RTM)) (filler) | 30.00 |
| Starch (disintegrant) | 46.00 |
| Orange oil (Orange Sweet (citrus sinensis oil), supplied by New Directions UK, | |
| Southampton, UK) | 1.25 |
| Tea-tree oil (Tea Tree Oil supplied by | |
| New Directions UK, UK) | 0.75 |

The ingredients were mixed together, granulated and fed into a die wherein the granules were compressed into a parallelopiped-shaped tablet having the following dimensions: length 5cm, width 2.5cm and height 2cm.

The granules were compressed in a die press having a force of 8 tons.

This composition has utility in treating an airspace within a container. The composition of Example 1 is particularly useful for containers in which there is a relatively large amount of moisture or moist contaminant present, which helps to release the tea-tree oil and orange oil into the airspace of the container by a wicking and/or evaporation action, in order to effectively mask any odours within the airspace due to the moisture or contaminants (via the orange oil) and to sterilise or effect biostatic action on microorganisms present (via the tea-tree oil).

### Example 2

The method of Example 1 was repeated for the following composition:

| | **% weight of the total weight of the composition** |
|---|---|
| Polyethylene glycol 6000 | 2.00 |
| Sodium chloride | 18.50 |
| Cellulose (filler) | 30.00 |
| Starch (disintergrant) | 46.00 |
| Sodium di-isopropylbenzene sulphonate | 1.50 |
| Lemon oil (Lemon Oil Pressed, supplied | |
| by New Directions UK, UK) | 1.25 |
| Tea-tree oil | 0.75 |

The composition, including the hygroscopic compound sodium di-isopropylbenzene sulphonate, is particularly suitable for containers which contain a relatively low amount of moisture or contain contaminated material that is relatively dry.

The combination of both a biocidal oil (tea-tree oil) and perfume oil (orange or lemon oil) in the tablets of Examples 1 and 2 make said tablets particularly effective in containers comprising biological waste, such as sanitary bins, sharps bins and body bags, for example. The biocidal oil helps to prevent harmful build-up of microorganims within the contaminated waste, and the airspace in the container; and the perfumed oil serves to mask any unpleasant odours emanating from the contaminated waste, and prevents the odours from exiting the container if and when it is opened.

Instead of tablets the compositions of Examples 1 and 2 may be formed into thin films, preferably having a thickness of less than 2mm, so that they may cover the bottom of a container or be wrapped around an article.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A sanitary composition for use in an airspace within a container, the sanitary composition comprising a filler and at least two oils, each oil having a sanitary property.

2. A sanitary composition as claimed in Claim 1 wherein the at least two oils are natural oils, synthetic oils or a combination of at least one natural oil and at least one synthetic oil.

3. A sanitary composition as claimed in Claims 1 and 2 wherein each oil is an aromatic oil.

4. A sanitary composition as claimed in Claim 2 wherein the or each natural oil is independently selected from tea tree oil, orange tree oil, citrus oil, jojoba oil, aloe vera oil, apricot oil, camellia oil, castor oil, coconut oil, rose hip oil, ambergris, apple blossom oil, jasmine oil, lilac oil, lavender oil, magnolia oil, orange blossom oil, oil of evening primrose, rose oil, wisteria oil, mentholyptus oil, and eucalyptus oil.

5. A sanitary composition as claimed in any preceding claim wherein at least one oil exhibits biocidal or biostatic properties.

6. A sanitary composition as claimed in any preceding claim wherein the composition comprises tea tree oil and at least one further oil.

7. A sanitary composition as claimed in Claim 6 wherein the further oil is a citrus oil.

8. A sanitary composition as claimed in any preceding claim wherein the composition further comprises a hygroscopic compound.

9. A sanitary composition as claimed in any preceding claim wherein the composition further comprises a lubricant.

10. A sanitary composition as claimed in Claim 9 wherein the lubricant is a polyglycol compound.

11. A sanitary composition as claimed in any preceding claim wherein the composition further comprises a sulphur-dioxide liberating compound.

12. A sanitary composition as claimed in any preceding claim wherein the filler is selected from cellulose, carboxymethyl cellulose, a diphosphate-containing compound, lactose, lactose monohydrate, dicalcium phosphate, and calcium sulphate.

13. A sanitary composition as claimed in any preceding claim wherein the filler is present in the composition in an amount of at least 10% w/w of the total weight of the composition.

14. A method of treating an airspace within a container, the method comprising locating a sanitary composition of any one of Claims 1 to 13 in the container.
